(19) Europäisches Patentamt
European Patent Office
Office européen des brevets

(11) Veröffentlichungsnummer: **0 074 608**
**B1**

(12) # EUROPÄISCHE PATENTSCHRIFT

(45) Veröffentlichungstag der Patentschrift: **03.07.85**

(21) Anmeldenummer: **82108238.5**

(22) Anmeldetag: **08.09.82**

(51) Int. Cl.⁴: **C 09 K 19/08**, C 09 K 19/12, C 07 C 121/75, C 07 C 121/60, C 07 C 43/225, C 07 C 43/192

(54) **Flüssigkristalline Halogenverbindungen, Verfahren zu ihrer Herstellung, diese enthaltende Dielektrika und elektrooptisches Anzeigeelement.**

(30) Priorität: **15.09.81 DE 3136624**

(43) Veröffentlichungstag der Anmeldung: **23.03.83 Patentblatt 83/12**

(45) Bekanntmachung des Hinweises auf die Patenterteilung: **03.07.85 Patentblatt 85/27**

(84) Benannte Vertragsstaaten: **AT CH DE FR IT LI NL SE**

(56) Entgegenhaltungen:
**EP - A - 0 014 840**
**EP - A - 0 023 728**
**EP - A - 0 051 738**
**FR - A - 2 208 673**
**GB - A - 1 433 130**

(73) Patentinhaber: **Merck Patent Gesellschaft mit beschränkter Haftung, Frankfurter Strasse 250, D-6100 Darmstadt (DE)**

(72) Erfinder: **Eidenschink, Rudolf, Dr., Erlenweg 17, D-6110 Dieburg (DE)**
Erfinder: **Römer, Michael, Dr., Im Grossen Garten 18, D-6054 Rodgau 2 (DE)**
Erfinder: **Weber, Georg, Wilhelm-Leuschner-Strasse 38, D-6101 Erzhausen (DE)**

**EP 0 074 608 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Für elektrooptische Anzeigeelemente werden in grossem Umfang die Eigenschaften nematischer oder nematisch-cholesterischer flüssigkristalliner Materialien ausgenutzt, ihre optischen Eigenschaften wie Lichtabsorption, Lichtstreuung, Doppelbrechung, Reflexionsvermögen oder Farbe unter dem Einfluss elektrischer Felder Signifikant zu verändern. Die Funktion derartiger Anzeigeelemente beruht dabei beispielsweise auf den Phänomenen der dynamischen Streuung, der Deformation aufgerichteter Phasen, dem Schadt-Helfrich-Effekt in der verdrillten Zelle oder dem cholesterisch-nematischen Phasenübergang.

Für die technische Anwendung dieser Effekte in elektronischen Bauelementen werden flüssigkristalline Dielektrika benötigt, die einer Vielzahl von Anforderungen genügen müssen. Besonders wichtig sind hier die chemische Beständigkeit gegenüber Feuchtigkeit, Luft und physikalischen Einflüssen wie Wärme, Strahlung im infraroten, sichtbaren und ultravioletten Bereich und elektrische Gleich- und Wechselfelder. Ferner wird von technisch verwendbaren flüssigkristallinen Dielektrika eine flüssigkristalline Mesophase im Temperaturbereich von mindestens 0° C bis +50° C, bevorzugt von −10° C bis 60° C, und eine möglichst niedrige Viskosität bei Raumtemperatur, die vorzugsweise nicht mehr als $70 \cdot 10^{-3}$ Pa.s betragen soll, gefordert. Schliesslich dürfen sie im Bereich des sichtbaren Lichtes keine Eigenabsorption aufweisen, d. h. sie müssen farblos sein.

Es ist bereits eine Anzahl von flüssigkristallinen Verbindungen bekannt, die den an Dielektrika für elektronische Bauelemente gestellten Stabilitätsanforderungen genügen und auch farblos sind. Hierzu gehören insbesondere die in der DE-OS Nr. 2139628 beschriebenen p,p'-disubstituierten Benzoesäurephenylester und die in der DE-OS Nr. 2636684 beschriebenen p,p'-disubstituierten Phenylcyclohexanderivate. In beiden genannten Verbindungsklassen wie auch in anderen Reihen von Verbindungen mit flüssigkristalliner Mesophase sind bisher keine Einzelverbindungen bekannt geworden, die in dem geforderten Temperaturbereich von 0 bis 60° C eine flüssigkristalline nematische Mesophase ausbilden. Es werden daher in der Regel Mischungen von zwei oder mehreren Verbindungen hergestellt, um als flüssigkristalline Dielektrika verwendbare Substanzen zu erhalten. Hierzu mischt man gewöhnlich mindestens eine Verbindung mit niedrigem Schmelz- und Klärpunkt mit einer anderen mit deutlich höherem Schmelz- und Klärpunkt. Hierbei wird normalerweise ein Gemisch erhalten, dessen Schmelzpunkt unter dem der niedriger schmelzenden Komponente liegt, während der Klärpunkt zwischen den Klärpunkten der Komponenten liegt. Trotzdem bereitet die Herstellung optimaler Dielektrika immer wieder Schwierigkeiten, da die Komponenten mit den hohen Schmelz- und Klärpunkten den Gemischen häufig auch eine hohe Viskosität verleihen. Dadurch werden die Schaltzeiten der damit hergestellten elektrooptischen Anzeigeelemente in unerwünschter Weise verlängert.

Ferner treten oft Probleme dadurch auf, dass die Löslichkeit der verschiedenen Komponenten ineinander, insbesondere bei Raumtemperatur oder tieferen Temperaturen, nur sehr begrenzt ist.

Der Erfindung liegt die Aufgabe zugrunde, flüssigkristalline Dielektrika herzustellen, die eine nematische Phase im geforderten Temperaturbereich aufweisen und in Flüssigkristallzellen bei Raumtemperatur ausreichend kurze Schaltzeiten ermöglichen.

Es wurde nun gefunden, dass die Halogenverbindungen der Formel (I):

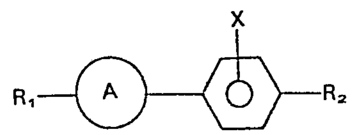

(I)

worin der Ring A 1,4-Phenylen oder trans-1,4-Cyclohexylen,

$R_1$ Alkyl oder Alkoxy mit 1 bis 12 Kohlenstoffatomen, oder, wenn A 1,4-Phenylen ist, auch CN,

$R_2$ Alkyl oder Alkoxy mit 1 bis 12 Kohlenstoffatomen, oder, wenn A trans-1,4-Cyclohexylen ist, auch Chlor oder Brom,

und X Fluor, Chlor oder Wasserstoff bedeutet, mit der Massgabe, dass mindestens einer der Substituenten $R_2$ und X ein Halogenatom ist, vorzüglich als Komponenten flüssigkristalliner Dielektrika geeignet sind. Dabei besitzen diese Verbindungen einen ausserordentlichen breiten Anwendungsbereich:

in Abhängigkeit von der Auswahl der Substituenten können die Verbindungen der Formel (I) sowohl als Basismaterialien dienen, aus denen flüssigkristalline Dielektrika zum überwiegenden Teil zusammengesetzt sind, es können aber auch Verbindungen der Formel (I) in geringeren Anteilen von beispielsweise 2 bis 45 Gewichtsprozent flüssigkristallinen Basismaterialien aus anderen Verbindungsklassen zugesetzt werden, um so Dielektrika mit einer verbreiterten flüssigkristallinen Mesophase oder einer niedrigeren Viskosität herzustellen oder die Grösse der dielektrischen Anisotropie eines solchen Dielektrikums zu beeinflussen.

Durch geeignete Auswahl der Substituenten $R_1$, $R_2$ und X lassen sich mit den Verbindungen der Formel (I) sowohl Dielektrika mit ausgeprägter positiver dielektrischer Anisotropie zur Verwendung in Anzeigeelementen auf der Basis der verdrillten nematischen Zelle oder des cholesterisch-nematischen Phasenübergangs herstellen, es können aber auch Dielektrika mit von Null nur wenig verschiedener oder auch mit negativer dielektrische Anisotropie hergestellt werden, die in Anzeigeelementen auf der Basis der dynamischen Streuung oder der Deformation aufgerichteter Phasen (DAP-Effekt) verwendet werden.

Die Verbindungen der Formel (I) sind in reinem Zustand farblos und bilden flüssigkristalline Mesophasen in einem erstaunlich breiten und für die elektrooptische Verwendung günstig gelegenen Temperaturbereich.

Gegenstand der Erfindung sind somit die flüssigkristallinen Halogenverbindungen der Formel (I), Verfahren zu ihrer Herstellung und ihre Verwendung als Komponenten flüssigkristalliner Dielektrika. Gegenstand der Erfindung sind weiterhin flüssigkristalline Dielektrika mit einem Gehalt an mindestens einer flüssigkristallinen Halogenverbindung der Formel (I) sowie elektrooptische Anzeigeelemente auf der Basis einer Flüssigkristallzelle, die ein derartiges flüssigkristallines Dielektrikum enthalten.

Die erfindungsgemässen flüssigkristallinen Halogenverbindungen der Formel (I) umfassen die Chlorphenylcyclohexan der Formel (Ia):

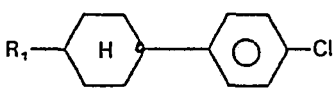
(Ia)

die fluorierten Biphenylderivate der Formel (Ib):

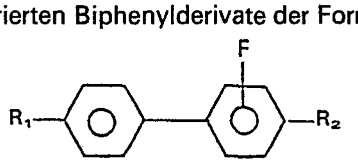
(Ib)

die Bromphenylcyclohexane der Formel (Ic):

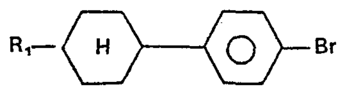
(Ic)

die chlorierten Biphenylderivate der Formel (Id):

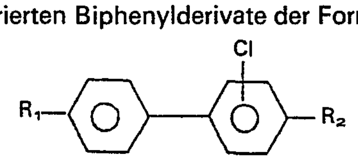
(Id)

die Fluorchlorphenylcyclohexane der Formel (Ie):

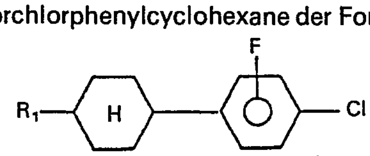
(Ie)

die Fluorbromphenylcyclohexane der Formel (If):

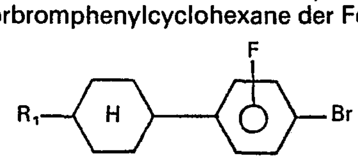
(If)

die Dichlorphenylcyclohexane der Formel (Ig):

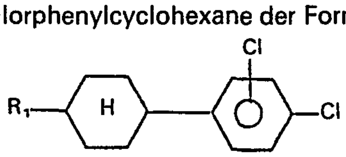
(Ig)

sowie die Chlorbromphenylcyclohexane der Formel (Ih):

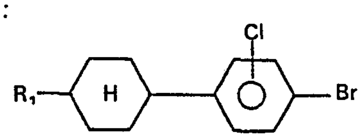
(Ih)

wobei die Substituenten $R_1$ und $R_2$ bei der Formel (I) angegebene Bedeutung besitzen. Soweit die flüssigkristallinen Halogenverbindungen der Formel (I) einen 1,4-disubstituierten Cyclohexanring enthalten, sind die Substituenten in den 1- und 4-Positionen zueinander transständig angeordnet; dies ist in den Formelbildern durch einen verstärkten schwarzen Punkt auf der rechten Seite des Rings kenntlich gemacht. Die Chlor- und Bromphenylcyclohexane der Formel (Ia) und (Ic) besitzen eine positive dielektrische Anisotropie und eine in einem günstigeren Temperaturbereich liegende nematische Mesophas auf als die analogen Fluorphenylcyclohexane nach der DE-OS Nr. 2907332. Die fluorierten bzw. chlorierten Biphenylderivate der Formeln (Ib) bzw. (Id) weisen eine positive dielektrische Anisotropie auf, wenn $R_1$ eine Cyanogruppe ist, und eine negative dielektrische Anisotropie, wenn $R_1$ Alkyl oder Alkoxy bedeutet. Auch bei diesen beiden Untergruppen der erfindungsgemässen Verbindungen bewirkt die Halogensubstitution eine deutliche Verbreiterung des nematischen Mesophasen-Temperaturbereichs sowie eine Verschiebung dieses Bereichs zu günstiger gelegenen Temperaturen. Die Dihalogenphenylcyclohexane der Teilformeln (Ie) bis (Ih) weisen je nach der Position und der Art des lateralen Halogensubstituenten eine mässige positive dielektrische Anisotropie (wenn der laterale Substituent dem terminalen benachbart ist) oder eine solche mit einem Wert um Null auf (wenn der laterale Substituent der Bindung zwischen den beiden Ringsystemen benachbart steht).

Unter den erfindungsgemässen Verbindungen der Formel (I) sind die der Teilformeln (Ia), (Ib), (Ic), (Id) und (Ig) bevorzugt, weil sie aus leichter zugänglichen Ausgangsmaterialien hergestellt werden können. Besonders gute Eigenschaften im Hinblick auf die Verwendung in Flüssigkristall-Anzeigeelementen besitzen darunter die Verbindungen der Teilformel (Ia), wenn $R_1$ Alkyl mit 2-12, vorzugsweise mit 3-8 C-Atomen ist, und der Teilformel (Ib).

In den Verbindungen der Formel (I) können die Alkyl- bzw. Alkoxyreste $R_1$ und $R_2$ geradkettig oder verzweigt sein. Wenn sie geradkettig sind, also Methyl, Ethyl, n-Propyl, n-Butyl- n-Pentyl, n-Hexyl, n-Heptyl, n-Octyl, n-Nonyl, n-Decyl- n-Undecyl oder n-Dodecyl oder die entsprechenden Alkoxygruppen bedeuten, besitzen die dadurch charakterisierten Verbindungen in der Regel höhere Klärpunkte als die mit verzweigten Flügelgruppen $R_1$ und/oder $R_2$. Deswegen enthält gewöhnlich höchstens eine der Flügelgruppen $R_1$ und $R_2$ eine verzweigte Kohlenstoffkette. Verbindungen der Formel (I) mit einer verzweigten Flügelgruppe $R_1$ oder $R_2$ sind gelegentlich wegen einer besseren Löslichkeit in den üblichen flüssigkristallinen Basismaterialien von Bedeutung, insbesondere aber als chirale Dotierstoffe, wenn sie durch die Kettenverzweigung optische Aktivität besitzen. Solche verzweigten Flügelgruppen enthalten nicht mehr als eine Kettenverzweigung. Bevorzugte verzeigte Kohlenwasserstoffreste R sind die, in denen sich an einer längeren Kohlenstoffkette in 1-, 2- oder 3-Stellung eine Methyl- oder Ethylgruppe befindet, beispielsweise 2-Methyl-propyl, 2-Methylbutyl, 3-Methylbutyl, 2-Methylpentyl, 3-Methylpentyl, 2-Ethylhexyl oder 1-Methylhexyl. Wenn $R_1$ und $R_2$ Alkyl oder Alkoxy bedeutet, können die Flügelgruppen $R_1$ und $R_2$ zusammen bis zu 24 Kohlenstoffatome enthalten. Im Rahmen der vorliegenden Erfindung sind darunter diejenigen bevorzugt, in denen $R_1$ und $R_2$ zusammen 3

bis 16, insbesondere von 4 bis 13 Kohlenstoffatome enthalten.

Die erfindungsgemässen Verbindungen werden in für derartige Substanzen üblicher Weise hergestellt. So werden beispielsweise die Verbindungen der Formeln (1a) bzw. (1c) erhalten, indem man ein Anilinderivat der Formel (II):

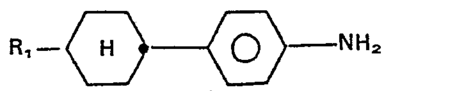 (II)

diazotiert und mit Kupfer(I)chlorid bzw. Kupfer(I)-bromid unter den Bedingungen einer Sandmeyer-Reaktion umsetzt. Die Diazotierung und die Sandmeyer-Reaktion werden dabei in an sich bekannte Weise ausgeführt, wie sie zum Beispiel in Gattermann-Wieland, Die Praxis des organischen Chemikers, 40. Auflage (Walter de Gruyter & Co., Berlin 1961), S. 252-254 beschrieben sind. Die Verbindungen der Formeln (Ib) und (Id), in denen $R_1$ Alkyl oder Alkoxy bedeutet, sowie die Verbindungen der Formeln (Ie), (If), (Ig) und (Ih) werden erhalten, indem eine entsprechende lateral nicht substituierte Verbindung der Formel (III):

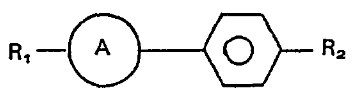 (III)

worin A und $R_2$ die in Formel (1) angegebene Bedeutung besitzen, zunächst zu einer Verbindung der Formel (IV) nitriert,

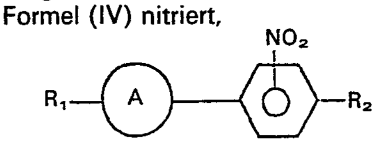 (IV)

die Nitroverbindung (IV) zur Aminoverbindung:

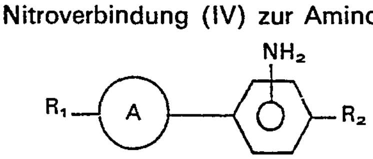 (V)

reduziert und diese nach Diazotierung mit Kupfer(I)-chlorid unter den Bedingungen einer Sandmeyer-Reaktion oder mit einem Tetrafluorborat unter den Bedingungen einer Schiemann-Balz-Reaktion umsetzt. Die Nitrierung von (III) zu (IV) wird dabei in für derartige Verbindungen üblicher Weise durchgeführt. Durch geeignete Wahl des Nitrierungsmittels, der Temperatur, der Reaktionsdauer und gegebenenfalls eines Lösungsmittels oder eines Katalysators kann die Bildung der möglichen isomeren Nitroverbindungen- in ortho- oder meta-Position zum Rest $R_2$ in Richtung auf ein erwünschtes Isomeres gesteuert werden; gegebenenfalls wird ein erhaltenes Gemisch der isomeren Nitroverbindungen in an sich bekannter Weise, zum Beispiel durch fraktionierte Kristallisation oder mit chromatografischen Methoden, aufgetrennt. Die Reduktion von (IV) zu (V) wird gleichfalls in literaturbekannter Weise durchgeführt, zum Beispiel durch katalytische Hydrierung, mit Zinn, Zink oder Eisen und Salzsäure, mit Zinn(II)-chlorid oder mit Dithionit. Die Diazotierung des Amins und die Sandmeyer-Reaktion zu den Chlorverbindungen (Id), (Ig) und (Ih) erfolgt wie die vorstehend beschriebene Synthese der Verbindungen (Ia) bzw. (Ic); die Schiemann-Balz-Synthese der Verbindungen (Ib), (Ic) und (If) aus dem entsprechenden Amin (5) kann nach einer der in „Organic Reactions", Band *5* (1949), S. 193-228 beschriebenen Verfahrensvarianten durchgeführt werden.

Die Herstellung solcher Verbindungen der Formel (I), worin A 1,4-Phenylen, $R_1$ CN, X Fluor oder Chlor und $R_2$ Alkyl oder Alkoxy bedeutet, erfolgt durch Nitrierung einer Verbindung der Formel:

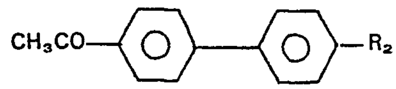 (VI)

zur Nitroverbindung:

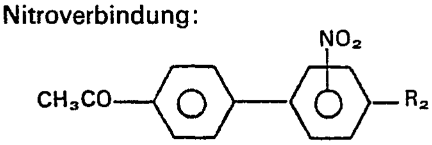 (VII)

deren Reduktion zur Aminoverbindung:

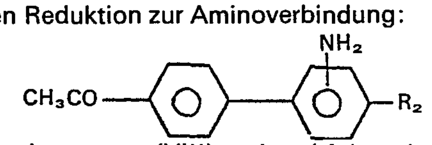 (VIII)

Diazotierung von (VIII) und nachfolgende Umsetzung mit Kupfer(I)chlorid unter den Bedingungen einer Sandmeyer-Reaktion oder mit Tetrafluorborat unter den Bedingungen einer Schiemann-Balz-Reaktion zur Halogenverbindung:

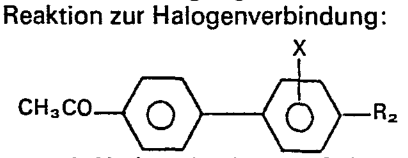 (IX)

die dann mit Hydroxylamin zum Oxim umgesetzt wird, aus dem durch eine an sich bekannte Beckmann-Umlagerung das Amin (X) hergestellt wird:

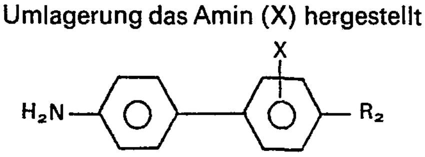 (X)

Dieses Amin (X) wird dann in an sich üblicher Weise diazotiert und unter den Bedingungen einer Sandmeyer-Reaktion mit Kupfer(I)-cyanid zum gewünschten Nitril umgesetzt.

Die erfindungsgemässen Dielektrika bestehen aus 2 bis 15, vorzugsweise 3 bis 12 Komponenten, darunter mindestens einer flüssigkristallinen Halogenverbindung der Formel (I).

Die anderen Bestandteile werden ausgewählt aus den nematischen oder nematogenen Substanzen aus den Klassen der Azoxybenzole, Benzylidenaniline, Biphenylene, Terphenyle, Phenyl- oder Cyclohexylbenzoate, Cyclohexancarbonsäure-phenyl- oder -cyclohexyl-ester, Phenylcyclohexane, Cyclohexylbiphenyle, Cyclohexylcyclohexane, Cyclohexylnaphthaline, 1,4-Bis-cyclohexylbenzole, 4,4'-Bis-cyclohexylbiphenyle, Phenyl- oder Cyclohexyl-pyrimidine, Phenyl- oder Cyclohexyldioxane, ggf. halogenierte Stilbene, Benzlphenylether, Tolane und substituierten Zimtsäure. Die wichtigsten als Bestandteile derartiger flüssigkristalliner Dielektrika in Frage kommenden Verbindungen lassen sich durch die Formel (XI) charakterisieren:

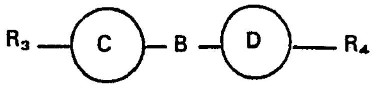 (XI)

worin C und D je ein carbo- oder heterocyclisches Ringsystem aus der aus 1,4-disubstituierten Benzol- und Cyclohexanringen, 4,4'-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituierten Naphthalin, Di- und Tetrahydronaphthalin, Chinazolin und Tetrahydrochinazolin gebildeten Gruppe,

B $-CH=CH-$ $-N(O)=N-$
$-CH=CY-$ $-CH=N(O)-$
$-\equiv C-$ $-CH_2-CH_2-$
$-CO-O-$ $-CH_2-O-$
$-CO-S-$ $-CH_2-S-$
$-CH=N-$ $-COO-$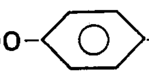$-COO-$

oder eine C–C-Einfachbindung,

Y Halogen, vorzugsweise Chlor, oder -CN, und $R_3$ und $R_4$ Alkyl, Alkoxy, Alkanoyloxy oder Alkoxycarbonyloxy mit bis zu 18, vorzugsweise bis zu 8 Kohlenstoffatomen, oder einer dieser Reste auch $-CN$, $-NC$, $-NO_2$, $-CF_3$, F, Cl oder Br bedeuten. Bei den meisten dieser Verbindungen sind $R_3$ und $R_4$ voneinander verschieden, wobei einer dieser Reste meist eine Alkyl- oder Alkoxygruppe ist. Aber auch andere Varianten der vorgesehenen Substituenten sind gebräuchlich. Viele solcher Substanzen oder auch Gemische davon sind im Handel erhältlich.

Die erfindungsgemässen Dielektrika enthalten in der Regel mindestens 30, vorzugsweise 50-99, insbesondere 60-98 Gewichtsteile der Verbindungen der Formel (I) und (II). Hiervon entfallen bevorzugt mindestens 5 Gewichtsteile, meist auch 10-40 Gewichtsteile auf eine oder mehrere Verbindungen der Formel (I). Jedoch werden von der Erfindung auch solche flüssigkristallinen Dielektrika umfasst, denen beispielsweise zu Dotierungszwecken nur weniger als 5 Gewichtsteile, zum Beispiel 0,1 bis 3 Gewichtsteile einer oder mehrerer Verbindungen der Formel (I) zugesetzt worden sind. Andererseits können die Verbindungen der Formel (I) bis zu 60 Gewichtsprozent der erfindungsgemässen Dielektrika ausmachen. Vorzugsweise enthalten die flüssigkristallinen Dielektrika nach der Erfindung 10 bis 30 Gewichtsprozent einer oder mehrerer Verbindungen der Formel (I).

Die Herstellung der erfindungsgemässen Dielektrika erfolgt in an sich üblicher Weise. In der Regel wird die gewünschte Menge der in geringerer Menge verwendeten Komponenten in der den Hauptbestandteil ausmachenden Komponente gelöst, zweckmässig bei erhöhter Temperatur. Wenn dabei eine Temperatur oberhalb des Klärpunktes des Hauptbestandteils gewählt wird, kann die Vollständigkeit des Lösevorgangs besonders leicht beobachtet werden.

Durch geeignete Zusätze können die flüssigkristallinen Dielektrika nach der Erfindung so modifiziert werden, dass sie in allen bisher bekannt gewordenen Arten von Flüssigkristallanzeigeelementen verwendet werden können. Derartige Zusätze sind dem Fachmann bekannt und sind in der einschlägigen Literatur ausführlich beschrieben. Beispielsweise können dichroitische Farbstoffe oder Substanzen zur Veränderung der dielektrischen Anisotropie, der Viskosität, der Leitfähigkeit und/oder der Orientierung der nematischen Phasen zugesetzt werden. Derartige Substanzen sind zum Beispiel in den DE-OS Nrn. 2209127, 2240864, 2321632, 2338281, 2450088, 2637430, 2853728 und 2902177 beschrieben.

Die folgenden Beispiele sollen die Erfindung erläutern, ohne sie zu begrenzen. In den Beispielen bedeuten F. den Schmelzpunkt und K. den Klärpunkt einer flüssigkristallinen Substanz in Grad Celsius; Siedetemperaturen sind mit Kp. bezeichnet. Wenn nichts anderes angegeben ist, bedeuten Angaben von Teilen oder Prozent Gewichtsteile bzw. Gewichtsprozent.

*Beispiel 1:*

189 g 4-(trans-4-n-Propylcyclohexyl)-anilinhydrochlorid werden in 500 ml 15%iger wässeriger Salzsäure suspendiert und hierzu bei −5°C innerhalb einer halben Stunde 300 ml einer 2,5 mol Natriumnitrit-Lösung gefügt. Diese Lösung wird in eine Kupfer-(I)-Salzlösung, die aus 250 g $CuSO_4 \cdot 5\,H_2O$, 88 g NaCl, 63 g $Na_2SO_3$, 1000 ml Wasser und 400 ml 32%iger Salzsäure hergestellt worden ist, bei 2°C innerhalb einer Stunde gegeben. Nach halbstündigem Erhitzen auf 40°C wird abgekühlt und die Lösung 3mal mit 300 ml Dichlormethan extrahiert. Die gesammelten organischen Phasen werden zweimal mit je 300 ml Wasser gewaschen.

Nach dem Abdestillieren des Lösungsmittels wird der Rückstand einer Destillation mit einer Drehbandkolonne im Vakuum unterzogen. Das bei 0,01 mmHg zwischen 105 und 120° übergehende 4-(trans-4-n-Propylcyclohexyl)-chlorbenzol wird aus Äthanol umkristallisiert; F. 36°, K. −17°.

Analog werden hergestellt:

4-(trans-4-Ethylcyclohexyl)-chlorbenzol,
4-(trans-4-n-Butylcyclohexyl)-chlorbenzol,
4-(trans-4-n-Pentylcyclohexyl)-chlorbenzol,
4-(trans-4-n-Hexylcyclohexyl)-chlorbenzol,
4-(trans-4-n-Heptylcyclohexyl)-chlorbenzol,
4-(trans-4-n-Octylcyclohexyl)-chlorbenzol,
4-(trans-4-n-Decylcyclohexyl)-chlorbenzol,
4-(trans-4-n-Dodecylcyclohexyl)-chlorbenzol,
4-[trans-4-(2-Methylbutyl)-cyclohexyl]-chlorbenzol,
4-[trans-4-(2-Ethylhexyl)-cyclohexyl]-chlorbenzol,
4-(trans-4-Ethylcyclohexyl)-brombenzol
4-(trans-4-n-Propylcyclohexyl)-brombenzol, F. 51°, K. 0°
4-(trans-4-n-Butylcyclohexyl)-brombenzol
4-(trans-4-n-Pentylcyclohexyl)-brombenzol
4-(trans-4-n-Hexylcyclohexyl)-brombenzol
4-(trans-4-n-Heptylcyclohexyl)-brombenzol

4-(trans-4-n-Octylcyclohexyl)-brombenzol
4-(trans-4-n-Decylcyclohexyl)-brombenzol
4-(trans-4-n-Dodecylcyclohexyl)-brombenzol
4-trans-4-(2-Methylbutyl)-cyclohexyl-brombenzol,
4-trans-4-(2-Ethylhexyl)-cyclohexyl-brombenzol.

*Beispiel 2:*

a) 37 g 4-n-Hexyloxy-4'-n-pentyl-biphenyl werden in 100 ml Dichlormethan gelöst und die Lösung unter intensivem Rühren bei 5° C mit 14 ml 65%iger Salpetersäure versetzt. Nach 1,5 stündigem Rühren wird das Gemenge in 500 ml Wasser geschüttet und die organische Phase abgetrennt. Nach mehrmaligem Waschen mit Waser wird das Dichlormethan abdestilliert. Der ölige Rückstand wird in 300 ml Methanol gelöst und unter Zugabe von 10 g Raney-Nickel bei Raumtemperatur unter Normaldruck hydriert, wobei die Wasserstoffaufnahme nach 1 stunde beendet ist. Nach dem Abfiltrieren des Katalysators wird das Methanol abdestilliert, der Rückstand in 25 ml Toluol gelöst und die Lösung über eine Chromatographiesäule geschickt (Kieselgel, Laufmittel Toluol). Die Hauptfraktion wird eingeengt und durch Destillation vom Lösungsmittel befreit. Dabei werden 20 g 3-Amino-4-n-hexyloxy-4'-n-pentyl-biphenyl, K. (smektisch-isotrop) 70°, erhalten.

b) 16,5 g 3-Amino-4-n-hexyloxy-4'-n-pentyl-biphenyl werden in 15 ml 36%iger wässeriger Salzsäure eingerührt. Nach Zugabe von 10 ml Dioxan wird bei 0° die Lösung von 3,0 g Natriumnitrit in 12 ml Wasser zugetropft. Unmittelbar darauf wird, ebenfalls bei 0°, eine Lösung von 15 g Natriumtetrafluorborat in 25 ml Wasser zugetropft. Der sich bildende Niederschlag wird nach 2 Stunden abfiltriert, mit Eiswasser gewaschen und utner vermindertem Druck bei Raumtemperatur getrocknet. Das pulvertrockene Diazoniumtetrafluoroborat wird bis zum Ende der Gasentwicklung auf 120° erhitzt und das zurückbleibende 3-Fluor-4-n-hexyloxy-4'-n-pentyl-biphenyl einer Kugelrohrdestillation im Vakuum einer Quecksilberdiffusionspumpe unterzogen; K. (smektisch-isotrop) 84°.

Analog werden hergestellt:
3-Fluor-4-methoxy-4'-n-propylbiphenyl,
3-Fluor-4-methoxy-4'-n-butylbiphenyl,
3-Fluor-4-methoxy-4'-n-pentylbiphenyl,
3-Fluor-4-methoxy-4'-n-hexylbiphenyl,
3-Fluor-4-methoxy-4'-n-heptylbiphenyl,
3-Fluor-4-methoxy-4'-n-octylbiphenyl,
3-Fluor-4-methoxy-4'-n-nonylbiphenyl,
3-Fluor-4-methoxy-4'-n-decylbiphenyl,
3-Fluor-4-methoxy-4'-n-undecylbiphenyl,
3-Fluor-4-methoxy-4'-n-dodecylbiphenyl,
3-Fluor-4-methoxy-4'-(2-methylbutyl)-biphenyl,
3-Fluor-4-ethoxy-4'-ethylbiphenyl,
3-Fluor-4-methoxy-4'-n-propylbiphenyl,
3-Fluor-4-methoxy-4'-n-butylbiphenyl,
3-Fluor-4-methoxy-4'-n-pentylbiphenyl,
3-Fluor-4-methoxy-4'-n-hexylbiphenyl,
3-Fluor-4-ethoxy-4'-n-heptylbiphenyl,
3-Fluor-4-ethoxy-4'-n-octylbiphenyl,
3-Fluor-4-ethoxy-4'-n-nonylbiphenyl,
3-Fluor-4-ethoxy-4'-n-decylbiphenyl,
3-Fluor-4-ethoxy-4'-n-undecylbiphenyl,
3-Fluor-4-ethoxy-4'-n-dodecylbiphenyl,
3-Fluor-4-ethoxy-4'-(2-methylbutyl)biphenyl,

3-Fluor-4-n-propyloxy-4'-methylbiphenyl,
3-Fluor-4-n-propyloxy-4'-ethylbiphenyl,
3-Fluor-4-n-propyloxy-4'-n-propylbiphenyl,
3-Fluor-4-n-propyloxy-4'-n-butylbiphenyl,
3-Fluor-4-n-propyloxy-4'-n-pentylbiphenyl,
3-Fluor-4-n-propyloxy-4'-n-hexylbiphenyl,
3-Fluor-4-n-propyloxy-4'-n-heptylbiphenyl,
3-Fluor-4-n-propyloxy-4'-n-octylbiphenyl,
3-Fluor-4-n-propyloxy-4'-n-nonylbiphenyl,
3-Fluor-4-n-propyloxy-4'-n-decylbiphenyl,
3-Fluor-4-n-propyloxy-4'-n-undecylbiphenyl,
3-Fluor-4-n-propyloxy-4'-n-dodecylbiphenyl,
3-Fluor-4-n-propyloxy-4'-(2-methylbutyl)-biphenyl,

3-Fluor-4-n-butyloxy-4'-methylbiphenyl,
3-Fluor-4-n-butyloxy-4'-ethylbiphenyl,
3-Fluor-4-n-butyloxy-4'-n-propylbiphenyl,
3-Fluor-4-n-butyloxy-4'-n-butylbiphenyl,
3-Fluor-4-n-butyloxy-4'-n-pentylbiphenyl,
3-Fluor-4-n-butyloxy-4'-n-hexylbiphenyl,
3-Fluor-4-n-butyloxy-4'-n-heptylbiphenyl,
3-Fluor-4-n-butyloxy-4'-n-octylbiphenyl,
3-Fluor-4-n-butyloxy-4'-n-nonylbiphenyl,
3-Fluor-4-n-butyloxy-4'-n-decylbiphenyl,
3-Fluor-4-n-butyloxy-4'-n-undecylbiphenyl,
3-Fluor-4-n-butyloxy-4'-n-dodecylbiphenyl,
3-Fluor-4-n-butyloxy-4'-(2-methylbutyl)-biphenyl,

3-Fluor-4-n-pentyloxy-4'-methylbiphenyl,
3-Fluor-4-n-pentyloxy-4'-ethylbiphenyl,
3-Fluor-4-n-pentyloxy-4'-n-propylbiphenyl,
3-Fluor-4-n-pentyloxy-4'-n-butylbiphenyl,
3-Fluor-4-n-pentyloxy-4'-n-pentylbiphenyl,
3-Fluor-4-n-pentyloxy-4'-hexylbiphenyl,
3-Fluor-4-n-pentyloxy-4'-n-heptylbiphenyl,
3-Fluor-4-n-pentyloxy-4'-n-octylbiphenyl,
3-Fluor-4-n-pentyloxy-4'-n-nonylbiphenyl,
3-Fluor-4-n-pentyloxy-4'-n-decylbiphenyl,
3-Fluor-4-n-pentyloxy-4'-n-undecylbiphenyl,
3-Fluor-4-n-pentyloxy-4'-(2-methylbutyl)-biphenyl,

3-Fluor-4-n-hexyloxy-4'-methylbiphenyl,
3-Fluor-4-n-hexyloxy-4'-ethylbiphenyl,
3-Fluor-4-n-hexyloxy-4'-n-propylbiphenyl,
3-Fluor-4-n-hexyloxy-4'-n-butylbiphenyl,
3-Fluor-4-n-hexyloxy-4'-n-hexylbiphenyl,
3-Fluor-4-n-hexyloxy-4'-n-heptylbiphenyl,
3-Fluor-4-n-hexyloxy-4'-n-octylbiphenyl,
3-Fluor-4-n-hexyloxy-4'-n-nonylbiphenyl,
3-Fluor-4-n-hexyloxy-4'-n-decylbiphenyl,
3-Fluor-4-n-hexyloxy-4'-(2-methylbutyl)-biphenyl,

3-Fluor-4-n-heptyloxy-4'-methylbiphenyl,
3-Fluor-4-n-heptyloxy-4'-ethylbiphenyl,

3-Fluor-4-n-heptyloxy-4'-n-propylbiphenyl,
3-Fluor-4-n-heptyloxy-4'-n-butylbiphenyl,
3-Fluor-4-n-heptyloxy-4'-n-pentylbiphenyl,
3-Fluor-4-n-heptyloxy-4'-n-hexylbiphenyl,
3-Fluor-4-n-heptyloxy-4'-n-heptylbiphenyl,
3-Fluor-4-n-heptyloxy-4'-n-octylbiphenyl,
3-Fluor-4-n-heptyloxy-4'-(2-methylbutyl)-biphenyl,

3-Fluor-4-n-octyloxy-4'-methylbiphenyl,
3-Fluor-4-n-octyloxy-4'-ethylbiphenyl,
3-Fluor-4-n-octyloxy-4'-n-propylbiphenyl,
3-Fluor-4-n-octyloxy-4'-n-butylbiphenyl,
3-Fluor-4-n-octyloxy-4'-n-pentylbiphenyl,
3-Fluor-4-n-octyloxy-4'-n-hexylbiphenyl,
3-Fluor-4-n-octyloxy-4'-n-heptylbiphenyl,
3-Fluor-4-n-octyloxy-4'-(2-methylbutyl)-biphenyl,

3-Fluor-4-(2-ethylhexyloxy)-4'-methylbiphenyl,
3-Fluor-4-(2-ethylhexyloxy)-4'-ethylbiphenyl,
3-Fluor-4-(2-ethylhexyloxy)-4'-n-propylbiphenyl,
3-Fluor-4-(2-ethylhexyloxy)-4'-n-butylbiphenyl,
3-Fluor-4-(2-ethylhexyloxy)-4'-n-pentylbiphenyl,
3-Fluor-4-(2-ethylhexyloxy)-4'-n-hexylbiphenyl,
3-Fluor-4-(2-ethylhexyloxy)-4'-n-heptylbiphenyl,
3-Fluor-4-(2-ethylhexyloxy)-4'-n-octylbiphenyl.

*Beispiel 3:*

a) 59 g 4,4'-Di-n-pentyl-biphenyl werden in fester Form in eine 40° warme Mischung aus 40 ml 65%iger Salpetersäure und 48 ml 96%iger Schwefelsäure eingetragen. Nach beendeter Zugabe wird das Gemisch noch 1 Stunde bei 60° gerührt und auf 600 g Eis gegossen. Das auskristallisierte 4,4'-Di-n-pentyl-2-nitro-biphenyl wird abfiltriert und aus Äthanol umkristallisiert.

b) 34 g 4,4'-Di-n-pentyl-2-nitro-biphenyl werden in 200 ml Tetrahydrofuran gelöst. Nach Zugabe von 3 g Palladium-Kohle (10% Pd) wird 1 Stunde lang bei Normaldruck und Raumtemperatur Wasserstoff eingeleitet. Anschliessend wird vom Katalysator abfiltriert und das Filtrat eingedampft. Das zurückbleibende 2-Amino-4,4'-di-n-pentyl-biphenyl wird aus Petroläther (Siedebereich 40-60°) umkristallisiert.

c) 15,5 g 2-Amino-4,4'-di-n-pentyl-biphenyl werden analog Beispiel 2 durch Umsetzung mit salpetriger Säure, Natriumtetrafluoroborat, Erhitzen und durch Hochvakuumdestillation und Umkristallisation aus Äthanol in 2-Fluor-4,4'-di-n-pentylbiphenyl überführt; F. −3°, K. −35°.

Analog werden hergestellt:

2-Fluor-4-methyl-4'-propylbiphenyl,
2-Fluor-4-methyl-4'-n-butylbiphenyl,
2-Fluor-4-methyl-4'-n-pentylbiphenyl,
2-Fluor-4-methyl-4'-n-hexylbiphenyl,
2-Fluor-4-methyl-4'-n-heptylbiphenyl,
2-Fluor-4-methyl-4'-n-octylbiphenyl,
2-Fluor-4-methyl-4'-n-nonylbiphenyl,
2-Fluor-4-methyl-4'-n-decylbiphenyl,
2-Fluor-4-methyl-4'-n-undecylbiphenyl,
2-Fluor-4-methyl-4'-n-dodecylbiphenyl,
2-Fluor-4-methyl-4'-(2-methylbutyl)-biphenyl,

2-Fluor-4-ethyl-4'-methylbiphenyl,
2-Fluor-4-ethyl-4'-ethylbiphenyl,
2-Fluor-4-ethyl-4'-n-propylbiphenyl,
2-Fluor-4-ethyl-4'-n-butylbiphenyl,
2-Fluor-4-ethyl-4'-n-pentylbiphenyl,
2-Fluor-4-ethyl-4'-n-hexylbiphenyl,
2-Fluor-4-ethyl-4'-n-heptylbiphenyl,
2-Fluor-4-ethyl-4'-n-octylbiphenyl,
2-Fluor-4-ethyl-4'-n-nonylbiphenyl,
2-Fluor-4-ethyl-4'-n-decylbiphenyl,
2-Fluor-4-ethyl-4'-n-undecylbiphenyl,
2-Fluor-4-ethyl-4'-n-dodecylbiphenyl,
2-Fluor-4-ethyl-4'-(2-methylbutyl)-biphenyl,

2-Fluor-4-n-propyl-4'-methylbiphenyl,
2-Fluor-4-n-propyl-4'-ethylbiphenyl,
2-Fluor-4-n-propyl-4'-n-propylbiphenyl,
2-Fluor-4-n-propyl-4'-n-butylbiphenyl,
2-Fluor-4-n-propyl-4'-n-pentylbiphenyl,
2-Fluor-4-n-propyl-4'-n-hexylbiphenyl,
2-Fluor-4-n-propyl-4'-n-heptylbiphenyl,
2-Fluor-4-n-propyl-4'-n-octylbiphenyl,
2-Fluor-4-n-propyl-4'-n-nonylbiphenyl,
2-Fluor-4-n-propyl-4'-n-decylbiphenyl,
2-Fluor-4-n-propyl-4'-n-undecylbiphenyl,
2-Fluor-4-n-propyl-4'-n-dodecylbiphenyl,
2-Fluor-4-n-propyl-4'-(2-methylbutyl)-biphenyl,

2-Fluor-4-n-butyl-4'-methylbiphenyl,
2-Fluor-4-n-butyl-4'-ethylbiphenyl,
2-Fluor-4-n-butyl-4'-n-propylbiphenyl,
2-Fluor-4-n-butyl-4'-n-butylbiphenyl,
2-Fluor-4-n-butyl-4'-n-pentylbiphenyl,
2-Fluor-4-n-butyl-4'-n-hexylbiphenyl,
2-Fluor-4-n-butyl-4'-n-heptylbiphenyl,
2-Fluor-4-n-butyl-4'-n-octylbiphenyl,
2-Fluor-4-n-butyl-4'-n-nonylbiphenyl,
2-Fluor-4-n-butyl-4'-n-decylbiphenyl,
2-Fluor-4-n-butyl-4'-n-undecylbiphenyl,
2-Fluor-4-n-butyl-4'-n-dodecylbiphenyl,
2-Fluor-4-n-butyl-4'-(2-methylbutyl)-biphenyl,

2-Fluor-4-n-pentyl-4'-methylbiphenyl,
2-Fluor-4-n-pentyl-4'-ethylbiphenyl,
2-Fluor-4-n-pentyl-4'-n-propylbiphenyl,
2-Fluor-4-n-pentyl-4'-n-butylbiphenyl,
2-Fluor-4-n-pentyl-4'-n-hexylbiphenyl,
2-Fluor-4-n-pentyl-4'-n-heptylbiphenyl,
2-Fluor-4-n-pentyl-4'-n-octylbiphenyl,
2-Fluor-4-n-pentyl-4'-n-nonylbiphenyl,
2-Fluor-4-n-pentyl-4'-n-decylbiphenyl,
2-Fluor-4-n-pentyl-4'-n-undecylbiphenyl,
2-Fluor-4-n-pentyl-4'-(2-methylbutyl)-biphenyl,

2-Fluor-4-n-hexyl-4'-methylbiphenyl,
2-Fluor-4-n-hexyl-4'-ethylbiphenyl,

2-Fluor-4-n-hexyl-4'-propylbiphenyl,
2-Fluor-4-n-hexyl-4'-n-butylbiphenyl,
2-Fluor-4-n-hexyl-4'-n-pentylbiphenyl,
2-Fluor-4-n-hexyl-4'-n-hexylbiphenyl,
2-Fluor-4-n-hexyl-4'-n-heptylbiphenyl,
2-Fluor-4-n-hexyl-4'-n-octylbiphenyl,
2-Fluor-4-n-hexyl-4'-n-nonylbiphenyl,
2-Fluor-4-n-hexyl-4'-n-decylbiphenyl,
2-Fluor-4-n-hexyl-4'-(2-methylbutyl)-biphenyl,

2-Fluor-4-n-heptyl-4'-methylbiphenyl,
2-Fluor-4-n-heptyl-4'-ethylbiphenyl,
2-Fluor-4-n-heptyl-4'-n-propylbiphenyl,
2-Fluor-4-n-heptyl-4'-n-butylbiphenyl,
2-Fluor-4-n-heptyl-4'-n-pentylbiphenyl,
2-Fluor-4-n-heptyl-4'-hexylbiphenyl,
2-Fluor-4-n-heptyl-4'-n-heptylbiphenyl,
2-Fluor-4-n-heptyl-4'-n-octylbiphenyl,
2-Fluor-4-n-heptyl-4'-(2-methylbutyl)-biphenyl,

2-Fluor-4-n-octyl-4'-methylbiphenyl,
2-Fluor-4-n-octyl-4'-ethylbiphenyl,
2-Fluor-4-n-octyl-4'-n-propylbiphenyl,
2-Fluor-4-n-octyl-4'-n-butylbiphenyl,
2-Fluor-4-n-octyl-4'-n-pentylbiphenyl,
2-Fluor-4-n-octyl-4'-n-hexylbiphenyl,
2-Fluor-4-n-octyl-4'-n-heptylbiphenyl,
2-Fluor-4-n-octyl-4'-(2-methylbutyl)-biphenyl.

*Beispiel 4:*

a) 15 g 4-n-Butyloxy-4'-n-pentylbiphenyl werden analog Beispiel 2a nitriert und anschliessend reduziert. Es werden 10 g 3-Amino-4-n-butyloxy-4'-n-pentylbiphenyl erhalten.

b) 10 g 3-Amino-4-n-butyloxy-4'-n-pentylbiphenyl werden analog Beispiel 1 in 3-Chlor-4-n-butyloxy-4'-n-pentylbiphenyl übergeführt; F. 38°, K. −40°.

Analog werden hergestellt:

3-Chlor-4-methoxy-4'-n-propylbiphenyl,
3-Chlor-4-ethoxy-4'-n-propylbiphenyl,
3-Chlor-4-n-propyloxy-4'-n-propylbiphenyl,
3-Chlor-4-n-butyloxy-4'-n-propylbiphenyl,
3-Chlor-4-n-pentyloxy-4'-n-propylbiphenyl,
3-Chlor-4-n-hexyloxy-4'-n-propylbiphenyl,
3-Chlor-4-n-heptyloxy-4'-n-propylbiphenyl,
3-Chlor-4-n-octyloxy-4'-n-propylbiphenyl,
3-Chlor-4-n-nonyloxy-4'-n-propylbiphenyl,
3-Chlor-4-n-decyloxy-4'-n-propylbiphenyl,
3-Chlor-4-n-propyloxy-4'-methylbiphenyl,
3-Chlor-4-n-butyloxy-4'-methylbiphenyl,
3-Chlor-4-n-pentyloxy-4'-methylbiphenyl,
3-Chlor-4-n-hexyloxy-4'-methylbiphenyl,
3-Chlor-4-n-heptyloxy-4'-methylbiphenyl,
3-Chlor-4-n-octyloxy-4'-methylbiphenyl,
3-Chlor-4-n-nonyloxy-4'-methylbiphenyl,
3-Chlor-4-n-decyloxy-4'-methylbiphenyl,
3-Chlor-4-ethoxy-4'-ethylbiphenyl,
3-Chlor-4-n-propyloxy-4'-ethylbiphenyl,
3-Chlor-4-n-butyloxy-4'-ethylbiphenyl,
3-Chlor-4-n-pentyloxy-4'-ethylbiphenyl,
3-Chlor-4-n-hexyloxy-4'-ethylbiphenyl,
3-Chlor-4-n-heptyloxy-4'-ethylbiphenyl,
3-Chlor-4-n-octyloxy-4'-ethylbiphenyl,
3-Chlor-4-n-nonyloxy-4'-ethylbiphenyl,
3-Chlor-4-n-decyloxy-4'-ethylbiphenyl,
3-Chlor-4-methoxy-4'-n-butylbiphenyl,
3-Chlor-4-ethoxy-4'-n-butylbiphenyl,
3-Chlor-4-n-propyloxy-4'-n-butylbiphenyl,
3-Chlor-4-n-butyloxy-4'-n-butylbiphenyl,
3-Chlor-4-n-pentyloxy-4'-n-butylbiphenyl,
3-Chlor-4-n-hexyloxy-4'-n-butylbiphenyl,
3-Chlor-4-n-heptyloxy-4'-n-butylbiphenyl,
3-Chlor-4-n-octyloxy-4'-n-butylbiphenyl,
3-Chlor-4-n-nonyloxy-4'-n-butylbiphenyl,
3-Chlor-4-n-decyloxy-4'-n-butylbiphenyl,
3-Chlor-4-methoxy-4'-n-pentylbiphenyl,
3-Chlor-4-ethoxy-4'-n-pentylbiphenyl,
3-Chlor-4-n-propyloxy-4'-n-pentylbiphenyl,
3-Chlor-4-n-butyloxy-4'-n-pentylbiphenyl,
3-Chlor-4-n-pentyloxy-4'-n-pentylbiphenyl,
3-Chlor-4-n-hexyloxy-4'-n-pentylbiphenyl,
3-Chlor-4-n-heptyloxy-4'-n-pentylbiphenyl,
3-Chlor-4-n-octyloxy-4'-n-pentylbiphenyl,
3-Chlor-4-methoxy-4'-n-hexylbiphenyl,
3-Chlor-4-ethoxy-4'-n-hexylbiphenyl,
3-Chlor-4-n-propyloxy-4'-n-hexylbiphenyl,
3-Chlor-4-n-butyloxy-4'-n-hexylbiphenyl,
3-Chlor-4-n-pentyloxy-4'-n-hexylbiphenyl,
3-Chlor-4-n-hexyloxy-4'-n-hexylbiphenyl,
3-Chlor-4-n-heptyloxy-4'-n-hexylbiphenyl,
3-Chlor-4-n-octyloxy-4'-n-hexylbiphenyl,
3-Chlor-4-methoxy-4'-n-heptylbiphenyl
3-Chlor-4-ethoxy-4'-n-heptylbiphenyl,
3-Chlor-4-n-propyloxy-4'-n-heptylbiphenyl,
3-Chlor-4-n-butyloxy-4'-n-heptylbiphenyl,
3-Chlor-4-n-pentyloxy-4'-n-heptylbiphenyl,
3-Chlor-4-n-hexyloxy-4'-n-heptylbiphenyl,
3-Chlor-4-methoxy-4'-n-octylbiphenyl,
3-Chlor-4-ethoxy-4'-n-octylbiphenyl,
3-Chlor-4-n-propyloxy-4'-n-octylbiphenyl,
3-Chlor-4-n-butyloxy-4'-n-octylbiphenyl,
3-Chlor-4-n-pentyloxy-4'-n-octylbiphenyl,
3-Chlor-4-n-hexyloxy-4'-n-octylbiphenyl.

*Beispiel 5:*

a) 26 g 4-(trans-4-n-Pentylcyclohexyl)-phenetol werden analog Beispiel 2a nitriert und anschliessend reduziert. Es werden 16 g 2-Amino-4-(trans-4-n-pentylcyclohexyl)-phenetol erhalten.

b) 14 g 2-Amino-4-(trans-4-n-pentylcyclohexyl)-phenetol werden analog Beispiel 2b diazotiert, mit Natriumtetrafluorborat umgesetzt und das Diazoniumtetrafluoborat thermisch zersetzt. Das zurückbleibende 2-Fluor-4-(trans-4-n-pentylcyclohexyl)-phenetol wird aus Ethanol umkristallisiert.

Analog werden hergestellt:

2-Fluor-4-(trans-4-methylcyclohexyl)-n-propyloxybenzol
2-Fluor-4-(trans-4-methylcyclohexyl)-n-butyloxybenzol
2-Fluor-4-(trans-4-methylcyclohexyl)-n-pentyloxybenzol
2-Fluor-4-(trans-4-methylcyclohexyl)-n-hexyloxybenzol

2-Fluor-4-(trans-4-methylcyclohexyl)-n-heptyloxybenzol
2-Fluor-4-(trans-4-methylcyclohexyl)-n-octyloxybenzol
2-Fluor-4-(trans-4-methylcyclohexyl)-n-decyloxybenzol
2-Fluor-4-(trans-4-methylcyclohexyl)-n-dodecyloxybenzol

2-Fluor-4-(trans-4-ethylcyclohexyl)-phenetol
2-Fluor-4-(trans-4-ethylcyclohexyl)-n-propyloxybenzol
2-Fluor-4-(trans-4-ethylcyclohexyl)-n-butyloxybenzol
2-Fluor-4-(trans-4-ethylcyclohexyl)-n-pentyloxybenzol
2-Fluor-4-(trans-4-ethylcyclohexyl)-n-hexyloxybenzol
2-Fluor-4-(trans-4-ethylcyclohexyl)-n-heptyloxybenzol
2-Fluor-4-(trans-4-ethylcyclohexyl)-n-octyloxybenzol
2-Fluor-4-(trans-4-ethylcyclohexyl)-n-decyloxybenzol
2-Fluor-4-(trans-4-ethylcyclohexyl)-n-dodecyloxybenzol
2-Fluor-4-(trans-4-ethylcyclohexyl)-(1-methylbutyloxy)-benzol

2-Fluor-4-(trans-4-n-propylcyclohexyl)-phenetol
2-Fluor-4-(trans-4-n-propylcyclohexyl)-n-propyloxybenzol
2-Fluor-4-(trans-4-n-propylcyclohexyl)-n-butyloxybenzol
2-Fluor-4-(trans-4-n-propylcyclohexyl)-n-pentyloxybenzol
2-Fluor-4-(trans-4-n-propylcyclohexyl)-n-hexyloxybenzol
2-Fluor-4-(trans-4-n-propylcyclohexyl)-n-heptyloxybenzol
2-Fluor-4-(trans-4-n-propylcyclohexyl)-n-octyloxybenzol
2-Fluor-4-(trans-4-n-propylcyclohexyl)-n-decyloxybenzol
2-Fluor-4-(trans-4-n-propylcyclohexyl)-n-dodecyloxybenzol

2-Fluor-4-(trans-4-n-butylcyclohexyl)-anisol
2-Fluor-4-(trans-4-n-butylcyclohexyl)-phenetol
2-Fluor-4-(trans-4-n-butylcyclohexyl)-n-propyloxybenzol
2-Fluor-4-(trans-4-n-butylcyclohexyl)-n-butyloxybenzol
2-Fluor-4-(trans-4-n-butylcyclohexyl)-n-pentyloxybenzol
2-Fluor-4-(trans-4-n-butylcyclohexyl)-n-hexyloxybenzol
2-Fluor-4-(trans-4-n-butylcyclohexyl)-n-heptyloxybenzol
2-Fluor-4-(trans-4-n-butylcyclohexyl)-n-octyloxybenzol
2-Fluor-4-(trans-4-n-butylcyclohexyl)-n-decyloxybenzol
2-Fluor-4-(trans-4-n-butylcyclohexyl)-n-dodecyloxybenzol
2-Fluor-4-(trans-4-n-butylcyclohexyl)-(1-methylbutyloxy)-benzol

2-Fluor-4-(trans-4-n-pentylcyclohexyl)-anisol
2-Fluor-4-(trans-4-n-pentylcyclohexyl)-n-propyloxybenzol
2-Fluor-4-(trans-4-n-pentylcyclohexyl)-n-butyloxybenzol
2-Fluor-4-(trans-4-n-pentylcyclohexyl)-n-pentyloxybenzol
2-Fluor-4-(trans-4-n-pentylcyclohexyl)-n-hexyloxybenzol
2-Fluor-4-(trans-4-n-pentylcyclohexyl)-n-heptyloxybenzol
2-Fluor-4-(trans-4-n-pentylcyclohexyl)-n-octyloxybenzol
2-Fluor-4-(trans-4-n-heptylcyclohexyl)-anisol
2-Fluor-4-(trans-4-n-heptylcyclohexyl)-phenetol
2-Fluor-4-(trans-4-n-heptylcyclohexyl)-n-propyloxybenzol
2-Fluor-4-(trans-4-n-heptylcyclohexyl)-n-butyloxybenzol
2-Fluor-4-(trans-4-n-heptylcyclohexyl)-n-pentyloxybenzol
2-Fluor-4-(trans-4-n-heptylcyclohexyl)-n-hexyloxybenzol
2-Fluor-4-(trans-4-n-heptylcyclohexyl)-n-heptyloxybenzol
2-Fluor-4-(trans-4-n-heptylcyclohexyl)-(1-methylbutyloxy)-benzol

Die folgenden Beispiele betreffen erfindungsgemässe Dielektrika mit einem Gehalt an mindestens einer Verbindung der Formel (I).

*Beispiel 6:*

Ein flüssigkristallines Dielektrikum aus:
20% trans-4-Propylcyclohexancarbonsäure-(4-ethoxy-phenyl)-ester,
20% trans-4-n-Butylcyclohexancarbonsäure-(4-ethoxyphenyl)-ester,
20% trans-4-n-Pentylcyclohexancarbonsäure-(4-methoxyphenyl)-ester,
30% 4-(trans-4-n-Propylcyclohexyl)-chlorbenzol, und
10% trans-4-n-Butylcyclohexancarbonsäure-[4-(trans-4-n-propylcyclohexyl)-phenyl]-ester
hat eine nematische Phase im Temperaturbereich von −3 bis +55°C, eine dielektrische Anisotropie von $\Delta\varepsilon = +0,9$ und eine erstaunlich niedrige Viskosität von nur $12 \cdot 10^{-3}$ Pa · s bei 20°.

*Beispiel 7:*

Ein flüssigkristallines Dielektrikum aus:
40% 4-n-Pentyl-4′-cyanobiphenyl,
30% 4-(trans-4-n-Pentylcyclohexyl)-chlorbenzol,
15% 4-(trans-4-n-Pentylcyclohexyl)-4′-ethylbiphenyl, und
15% 4-(trans-4-n-Propylcyclohexyl)-4′-ethylbiphenyl
hat eine nematische Phase im Temperaturbereich

von −7° bis +54°, eine dielektrische Anisotropie von Δε = +6,7, eine optische Anisotropie von Δn = 0,18 und eine Viskosität von nur $14 \cdot 10^{-3}$ Pa · s bei 20°. Aufgrund dieser niedrigen Viskosität und der vergleichsweise hohen optischen und dielektrischen Anisotropie ist es gut geeignet für Flüssigkristall-Anzeigeelemente auf der Basis der verdrillten nematischen Zelle.

*Beispiel 8:*

Ein flüssigkristallines Dielektrikum aus:
27% 4-(trans-4-n-Propylcyclohexyl)-chlorbenzol,
18% trans-4-n-Propylcyclohexancarbonsäure-(4-ethoxyphenyl)-ester,
18% trans-4-n-Butylcyclohexancarbonsäure-(4-ethoxyphenyl)-ester,
17% trans-4-n-Butylcyclohexancarbonsäure-[4-(trans-4-n-propylcyclohexyl)-phenyl]-ester,
10% 2-(4-Cyanophenyl)-5-n-propyl-1,3-dioxan, und
10% 2-(4-Cyanophenyl)-5-n-butyl-1,3-dioxan
hat eine nematische Phase im Temperaturbereich von −6° bis +57°, eine dielektrische Anisotropie von Δε = +7 und eine Viskosität von nur $16 \cdot 10^{-3}$ Pa · s. Dieses Dielektrikum weist in Flüssigkristallanzeigelementen auf der Basis der verdrillten nematischen Zelle eine steile Kennlinie auf und ist daher gut geeignet für im Multiplexbetrieb angesteuerte Anzeigen.

*Beispiel 9:*

Ein flüssigkristallines Dielektrikum aus:
30% 4-n-Pentyl-4′-cyanobiphenyl,
25% 4-(trans-4-n-Butylcyclohexyl)-chlorbenzol,
18% trans-4-n-Propylcyclohexyl-benzonitril,
10% 4-(trans-4-n-Pentylcyclohexyl)-4′-(trans-4-n-propylcyclohexyl)-biphenyl,
9% 4-(trans-4-n-Pentylcyclohexyl)-4′-cyanobiphenyl, und
8% 4-n-Pentyl-4″-cyanoterphenyl
hat eine nematische Phase im Temperaturbereich von −9° bis +75°, eine dielektrische Anisotropie Δε = +9,4, eine optische Anisotropie von Δn + 0,18 und eine Viskosität von nur $21 \cdot 10^{-3}$ Pa · s bei 20°. Es ist gut geeignet für grossflächige, schnell schaltende Anzeigeelemente auf der Basis der verdrillten nematischen Zelle.

*Beispiel 10:*

Ein flüssigkristallines Dielektikum aus:
27% 4-(trans-4-n-Heptylcyclohexyl)-chlorbenzol,
23% 4-(trans-4-n-Pentylcyclohexyl)-2′-fluor-4′-ethylbiphenyl,
22% 4-(trans-4-n-Pentylcyclohexyl)-4′-ethylbiphenyl,
18% 4-(trans-4-n-Propylcyclohexyl)-benzonitril, und
10% 4-(trans-4-n-Pentylcyclohexyl)-4′-(trans-4-n-propylcyclohexyl)-biphenyl
hat eine nematische Phase im Temperaturbereich von −9° bis +95°, eine dielektrische Anisotropie von Δε= +3,8 und eine Viskosität von $19 \cdot 10^{-3}$ Pa · s bei 20°, die auch bei 0° auf nicht mehr als $55 \cdot 10^{-3}$ ansteigt. Dieses Dielektrikum ist für unter extremen Temperaturbedingungen betriebene Flüssigkristallanzeigeelemente gut geeignet.

*Beispiel 11:*

Ein flüssigkristallines Dielektrikum aus:
15% 4-(trans-4-n-Propylcyclohexyl)-benzonitril,
11% 4-(trans-4-n-Butylcyclohexyl)-benzonitril,
21% 4-(trans-4-n-Pentylcyclohexyl)-benzonitril,
21% 4-(trans-4-n-Pentylcyclohexyl)-4′-ethylbiphenyl,
4% 4-(trans-4-n-Pentylcyclohexyl)-4′-cyanobiphenyl,
12% 4-(trans-4-n-Pentylcyclohexyl)-4′-(trans-4-n-propylcyclohexyl)-biphenyl, und
16% 4,4′-Bis-n-pentyl-2-fluorbiphenyl
hat eine nematische Phase im Temperaturbereich von −20° bis +90°, eine dielektrische Anisotropie von Δε = 10,5 und eine Viskosität von nur $20 \cdot 10^{-3}$ Pa · s. Es eignet sich besonders für den Einsatz in Anzeigeelementen auf der Basis der verdrillten nematischen Zelle unter extremen Temperaturbedingungen.

*Beispiel 12:*

Ein flüssigkristallines Dielektrikum aus:
25% 4-(trans-4-Ethylcyclohexyl)-chlorbenzol,
20% 4-(trans-4-n-Propylcyclohexyl)-phenetol,
15% 4-(trans-4-n-Propylcyclohexyl)-ethylbenzol,
15% 4-(trans-4-n-Propylcyclohexyl)-4′-ethylbiphenyl,
15% 4-(trans-4-n-Pentylcyclohexyl)-4′-ethylbiphenyl, und
10% 4-(trans-4-n-Pentylcyclohexyl)-4′-(trans-4-n-propylcyclohexyl)-biphenyl
hat eine nematische Phase im Temperaturbereich von −13° bis +62° und eine Viskosität von nur $9 \cdot 10^{-3}$ Pa · s bei 20°.

*Beispiel 13:*

Ein flüssigkristallines Dielektrikum aus:
27% 4-(trans-4-n-Propylcyclohexyl)-chlorbenzol,
18% 4-(trans-4-n-Propylcyclohexyl)-benzonitrol,
16% 4-(trans-4-n-Propylcyclohexyl)-phenetol,
22% 4-(trans-4-n-Pentylcyclohexyl)-4′-ethylbiphenyl,
7% 4-(trans-4-n-Pentylcyclohexyl)-4′-cyanobiphenyl, und
10% 4-(trans-4-n-Pentylcyclohexyl)-biphenyl
hat eine nematische Phase im Temperaturbereich von −15° bis +86° und eine Viskosität von nur $15 \cdot 10^{-3}$ Pa · s bei 20°; auch bei 0° steigt die Viskosität nur auf $48 \cdot 10^{-3}$ an.

## Patentansprüche

1. Flüssigkristalline Halogenverbindungen der Formel (I):

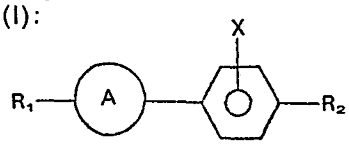

(I)

worin der Ring A 1,4-Phenylen oder trans-1,4-Cyclo-hexylen,

$R_1$ Alkyl oder Alkoxy mit 1 bis 12 Kohlenstoffatomen, oder, wenn A 1,4-Phenylen ist, auch CN,

$R_2$ Alkyl oder Alkoxy mit 1 bis 12 Kohlenstoffatomen, oder wenn A trans-1,4-Cyclohexylen ist, auch Chlor oder Brom, und

X Fluor, Chlor oder Wasserstoff bedeutet, mit der Massgabe, dass mindestens einer der Substituenten $R_2$ und X ein Halogenatom ist.

2. Flüssigkristalline Halogenverbindungen nach Anspruch 1 der Formel (Ia):

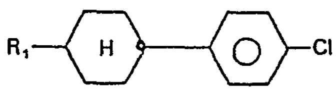

(Ia)

worin $R_1$ Alkyl mit 2 bis 12 Kohlenstoffatomen bedeutet.

3. Flüssigkristalline Halogenverbindungen nach Anspruch 1 der Formel (Ib):

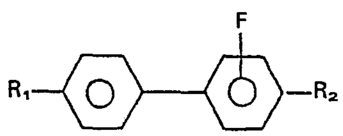

(Ib)

worin $R_1$ und $R_2$ die in Anspruch 1 angegebene Bedeutung besitzen.

4. Verwendung der flüssigkristallinen Halogenverbindungen der Formel (I) nach Anspruch 1 als Komponenten flüssigkristalliner Dielektrika für elektrooptische Anzeigeelemente.

5. Flüssigkristallines Dielektrikum mit mindestens 2 flüssigkristallinen Komponenten, dadurch gekennzeichnet, dass mindestens eine dieser Komponenten eine flüssigkristalline Halogenverbindung der Formel (I) nach Anspruch 1 ist.

6. Flüssigkristallines Dielektrikum nach Anspruch 5, dadurch gekennzeichnet, dass es 5-40 Gewichtsprozent mindestens einer Verbindung der Formel (I) enthält.

7. Elektrooptisches Anzeigeelement auf der Basis einer Flüssigkristallzelle, dadurch gekennzeichnet, dass die Flüssigkristallzelle ein Dielektrikum nach Anspruch 5 enthält.

8. Verfahren zur Herstellung der flüssigkristallinen Halogenverbindungen der Formel (I):

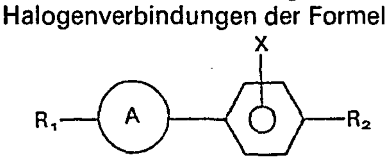

(I)

worin der Ring A 1,4-Phenylen oder trans-1,4-Cyclohexylen,

$R_1$ Alkyl oder Alkoxy mit 1 bis 12 Kohlenstoffatomen, oder, wenn A 1,4-Phenylen ist, auch CN,

$R_2$ Alkyl oder Alkoxy mit 1 bis 12 Kohlenstoffatomen, oder, wenn A trans-1,4-Cyclohexylen ist, auch Chlor oder Brom, und

X Fluor, Chlor oder Wasserstoff bedeutet, mit der Massgabe, dass mindestens einer der Substituenten $R_2$ und X ein Halogenatom ist, dadurch gekennzeichnet, dass man

a) zur Herstellung einer Verbindung der Formel (I), worin der Ring A trans-1,4-Cyclohexylen, $R_1$ Alkyl oder Alkoxy, $R_2$ Chlor oder Brom und Wasserstoff bedeutet, ein Anilinderivat der Formel (II)

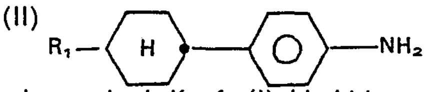

(II)

diazotiert und mit Kupfer(I)chlorid bzw. Kupfer(I)-bromid unter den Bedingungen einer Sandmeyer-Reaktion umsetzt, oder

b) zur Herstellung einer Verbindung der Formel (I), worin $R_1$ Alkyl oder Alkoxy und X Fluor oder Chlor bedeuten, und A und $R_2$ die vorstehend genannte Bedeutung besitzen, eine Verbindung der Formel (III):

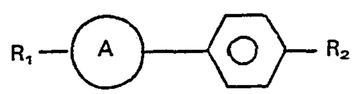

(III)

nitriert, die erhaltene Nitroverbindung der Formel (IV):

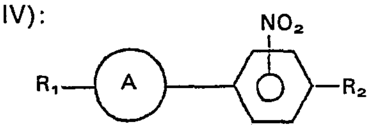

(IV)

zur Aminoverbindung (V) reduziert

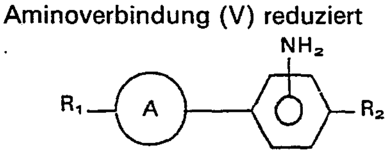

(V)

und diese nach Diazotierung mit Kupfer(I)chlorid unter den Bedingungen einer Sandmeyer-Reaktion oder mit Tetrafluoborat unter den Bedingungen einer Schiemann-Balz-Reaktion umsetzt, oder

c) zur Herstellung einer Verbindung der Formel (I), worin A 1,4-Phenylen, $R_1$ CN und X Fluor oder Chlor bedeuten, und $R_2$ die vorstehend angegebene Bedeutung besitzt, eine Verbindung der Formel (VI):

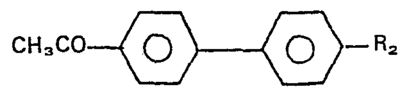

(VI)

nitriert, die erhaltene Nitroverbindung der Formel (VII):

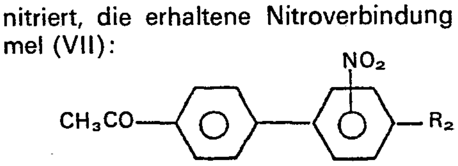

(VII)

zur Aminoverbindung (VIII) reduziert,

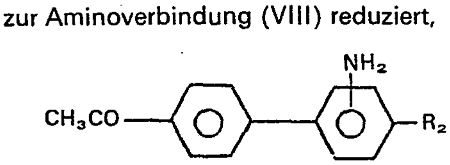

(VIII)

diese nach Diazotierung mit Kupfer(I)chlorid unter den Bedingungen einer Sandmeyer-Reaktion oder mit Tetrafluoborat unter den Bedingungen einer Schiemann-Balz-Reaktion zur Halogenverbindung (IX) umsetzt

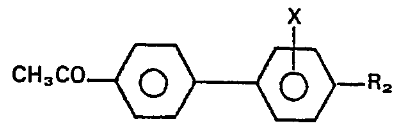

(IX)

und nach Umsetzung mit Hydroxylamin und einer Beckmann-Umlagerung zum Amin (X):

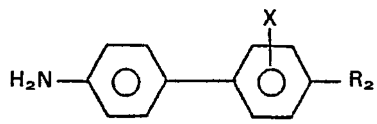

(X)

dieses nach Diazotierung mit Kupfer(I)cyanid unter den Bedingungen einer Sandmeyer-Reaktion umsetzt.

## Claims

1. Liquid-crystalline halogen compounds of the formula (I):

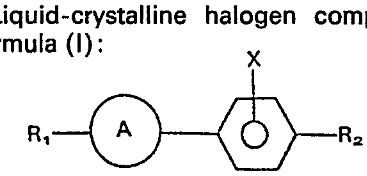

(I)

wherein the ring A is 1,4-phenylene or trans-1,4-cyclohexylene,

$R_1$ is alkyl or alkoxy having 1 to 12 carbon atoms or, if A is 1,4-phenylene, is also cyano,

$R_2$ is alkyl or alkoxy having 1 to 12 carbon atoms or, if A is 1,4-cyclohexylene, is also chlorine or bromine and X is fluorine, chlorine or hydrogen, with the proviso that at least one of the substituents $R_2$ and X is a halogen atom.

2. Liquid-crystalline halogen compounds according to Claim 1, of the formula (Ia):

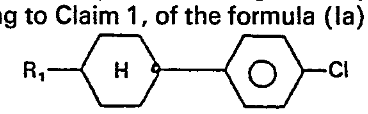

(Ia)

wherein $R_1$ is alkyl having 2 to 12 carbon atoms.

3. Liquid-crystalline halogen compounds according to Claim 1, of the formula (Ib):

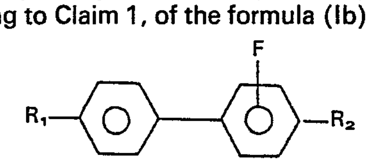

(Ib)

wherein $R_1$ and $R_2$ have the meanings given in Claim 1.

4. Use of the liquid-crystalline halogen compounds of the formula (I) according to Claim 1 as components of liquid-crystalline dielectrics for electro-optical display elements.

5. Liquid-crystalline dielectric with at least 2 liquid-crystalline components, characterised in that at least one of these components is a liquid-crystalline halogen compound of the formula (I) according to Claim 1.

6. Liquid-crystalline dielectric according to Claim 5, characterised in that it contains 5-40 per cent by weight of at least one compound of the formula (I).

7. Electro-optical display element based on a liquid crystal cell, characterised in that the liquid crystal cell contains a dielectric according to Claim 5.

8. Process for the preparation of the liquid-crystalline halogen compounds of the formula (I):

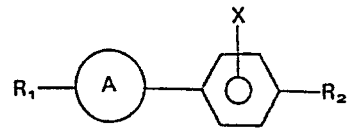

(I)

wherein the ring A is 1,4-phenylene or trans-1,4-cyclohexylene,

$R_1$ is alkyl or alkoxy having 1 to 12 carbon atoms, or if A is 1,4-phenylene, is also CN,

$R_2$ is alkyl or alkoxy having 1 o 12 carbon atoms or, if A is 1,4-cyclohexylene, is also chlorine or bromine and X is fluorine, chlorine or hydrogen, with the proviso that at least one of the substituents $R_2$ or X is a halogen atom, characterised in that

a) for the preparation of a compound of the formula (I), wherein the ring A is trans-1,4-cyclohexylene, $R_1$ is alkyl or alkoxy, $R_2$ is chlorine or bromine and X is hydrogen, an aniline derivative of the formula (II):

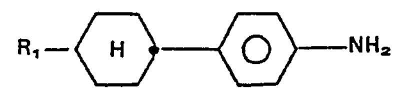

(II)

is diazotised and reacted with copper(I) chloride or copper(I) bromide under the conditions of the Sandmeyer reaction, or

b) for the preparation of a compound of the formula (I) wherein $R_1$ is alkyl or alkoxy, X is fluorine or chlorine and A and $R_2$ have the meaning given above, a compound of the formula (III):

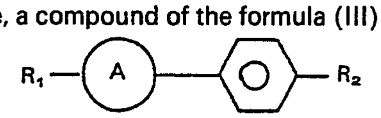

(III)

is nitrated, the resulting nitro compound of the formula (IV):

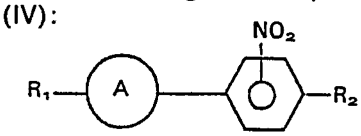

(IV)

is reduced to the amino compound (V):

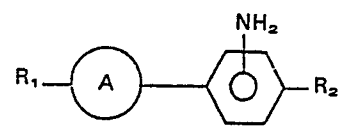

(V)

and the latter, after diazotisation, is reacted with copper(I) chloride under the conditions of a Sandmeyer reaction or with tetrafluoborate under the conditions of a Schiemann-Balz reaction, or

c) for the preparation of a compound of the formula (I), wherein A is 1,4-phenylene, $R_1$ is CN, X is fluorine or chlorine and $R_2$ has the meaning given above, a compound of the formula (VI):

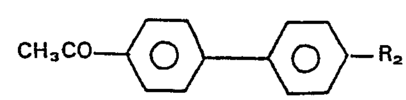

(VI)

is nitrated, the resulting nitro compound of the formula (VII):

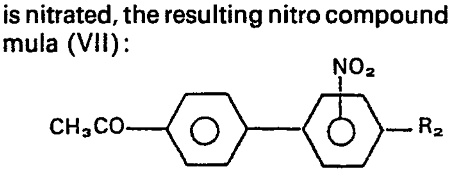

(VII)

is reduced to the amino compound (VIII):

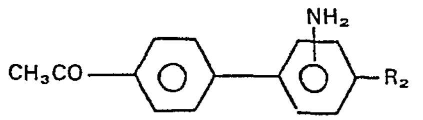

(VIII)

the latter, after diazotisation, is reacted with copper(I) chloride under the conditions of a Sandmeyer reaction or with tetrafluoborate under the conditions of a Schiemann-Balz reaction to give the halogen compound (IX):

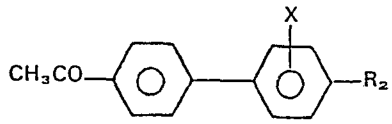

(IX)

and, after reaction with hydroxylamine and a Beckann rearrangement to give the amine (X):

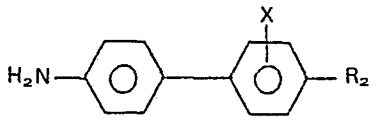

(X)

the latter, after diazotisation, is reacted with copper(I) cyanide under the conditions of a Sandmeyer reaction.

## Revendications

1. Composés halogénés à cristaux liquides de formule (I):

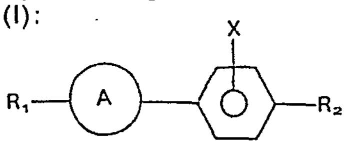

(I)

dans laquelle le noyau A est un noyau 1,4-phénylène ou trans-1,4-cyclohexylène,

$R_1$ représente un groupe alkyle ou alcoxy en C 1-C 12 ou bien encore, lorsque le noyau A est un noyau 1,4-phénylène, le groupe CN,

$R_2$ représente un groupe alkyle ou alcoxy en C 1-C 12, ou bien encore, lorsque A représente un noyau trans-1,4-cyclohexylène, le chlore ou le brome, et

X représente le fluor, le chlore ou l'hydrogène, étant spécifié que l'un au moins des substituants $R_2$ et X est un atome d'halogène.

2. Composés halogénés à cristaux liquides selon la revendication 1, de formule (Ia):

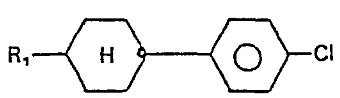

(Ia)

dans laquelle $R_1$ représente un groupe alkyle en C 2-C 12.

3. Composés halogénés à cristaux liquides selon la revendication 1, de formule (Ib):

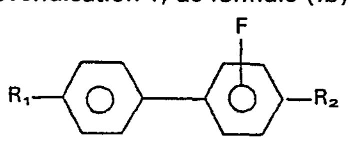

(Ib)

dans laquelle $R_1$ et $R_2$ ont les significations indiquées dans la revendication 1.

4. Utilisation des composés halogénés à cristaux liquides de formule (I) selon la revendication 1 en tant que composants de diélectriques à cristaux liquides pour éléments d'affichage électro-optiques.

5. Diélectrique à cristaux liquides contenant au moins 2 composants à cristaux liquides, caractérisé en ce que l'un au moins de ces composants est un composé halogéné à cristaux liquides de formule (I) selon la revendication 1.

6. Diélectrique à cristaux liquides selon la revendication 5, caractérisé en ce qu'il contient de 5 à 40% en poids d'au moins un composé de formule (I).

7. Eléments d'affichage électro-optiques à base d'une cellule à cristaux liquides, caractérisés en ce que la cellule à cristaux liquides contient un diélectrique selon la revendication 5.

8. Procédé de préparation des composés halogénés à cristaux liquides de formule (I):

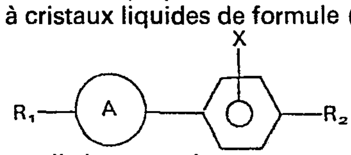

(I)

dans laquelle le noyau A est un noyau 1,4-phénylène ou trans-1,4-cyclohexylène,

$R_1$ représente un groupe alkyle ou alcoxy en C 1-C 12, ou bien encore, lorsque le noyau A est un noyau 1,4-phénylène, le groupe CN,

$R_2$ représente un groupe alkyle ou alcoxy en C 1-C 12, ou bien encore, lorsque le noyau A est un noyau trans-1,4-cyclohexylène, le chlore ou le brome, et

X représente le fluor, le chlore ou l'hydrogène, étant spécifié que l'un au moins des substituants $R_2$ et X est un atome d'halogène,

caractérisé en ce que:

a) pour préparer un composé de formule (I) dans laquelle le noyau A est un noyau trans-1,4-cyclohexylène, $R_1$ représente un groupe alkyle ou alcoxy, $R_2$ le chlore ou le brome et X l'hydrogène, on diazote un dérivé de l'aniline répondant à la formule (II):

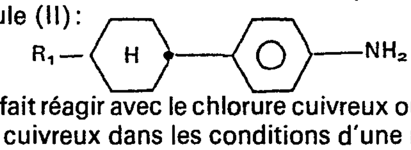

(II)

et on fait réagir avec le chlorure cuivreux ou le bromure cuivreux dans les conditions d'une réaction de Sandmeyer, ou bien

b) pour préparer un composé de formule (I) dans laquelle $R_1$ représente un groupe alkyle ou alcoxy et X représente le fluor ou le chlore, A et $R_2$ ayant les significations indiquées ci-dessus, on nitre un composé de formule (III):

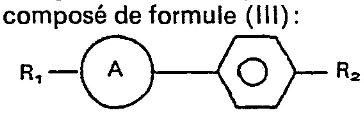

(III)

ce qui donne un composé nitré de formule (IV):

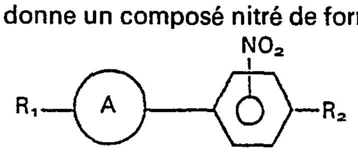

(IV)

qu'on réduit en un composé aminé (V):

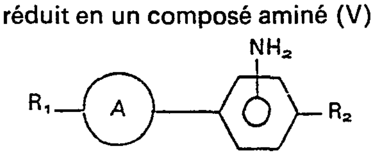

(V)

qu'on fait réagir après diazotation avec du chlorure cuivreux dans les conditions d'une réaction de Sandmeyer ou avec un tétrafluoborate dans les conditions d'une réaction de Schiemann-Balz, ou bien

c) pour préparer un composé de formule (I) dans laquelle A représente un noyau 1,4-phénylène, $R_1$ représente le groupe CN et X représente le fluor ou le chlore, $R_2$ ayant les significations indiquées ci-dessus, on nitre un composé de formule (VI):

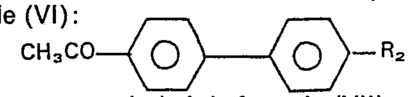
(VI)

en un composé nitré de formule (VII):

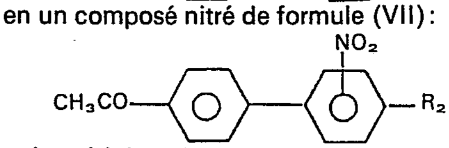
(VII)

qu'on réduit en le composé aminé (VIII):

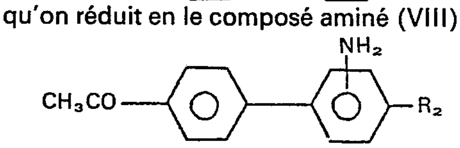
(VIII)

qu'on fait réagir après diazotation avec du chlorure cuivreux dans les conditions d'une réaction de Sandmeyer ou avec un tétrafluoborate dans les conditions d'une réaction de Schiemann-Balz, ce qui donne le composé halogéné (IX):

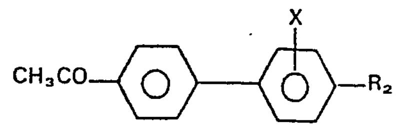
(IX)

qu'on fait réagir avec l'hydroxylamine en convertissant ensuite par une transposition de Beckmann en l'amine (X):

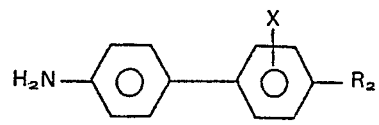
(X)

qu'on fait réagir après diazotation avec du cyanure cuivreux dans les conditions d'une réaction de Sandmeyer.